# EUROPÄISCHE PATENTANMELDUNG

(11) **EP 2 090 304 A2**
(43) Veröffentlichungstag der Anmeldung: **19.08.2009**
(21) Anmeldenummer: 09075090.2
(22) Anmeldetag: 17.01.2002
(51) Int. Cl.: A61K 31/195, A61K 31/198, A61K 31/401, A61K 31/42, A61K 31/661, A61K 31/6615, A61K 31/7024, A61P 35/00, A61P 37/06, A61P 29/00

(54) **Verwendung von Zuckerphosphaten, Zuckerphosphatanalogen, Aminosäuren und/oder Aminosäureanalogen zur Modulation des Glykose-Enzym-Komplexes, des Malat Aspartat Shuttles und/oder der Transaminasen**

(30) Priorität: 13.03.2001 DE 10112926
(62) Teilanmeldung aus: 02704608.5
(71) Anmelder: ScheBo Biotech AG, 35394 Giessen (DE)
(72) Erfinder: Eigenbrodt, Erich, 35440 Linden/Grossen Linden (DE); Mazurek, Sybille, 35440 Linden (DE); Grimm, Helmut, 35394 Giessen (DE)
(74) Vertreter: Jungblut, Bernhard Jakob

(57) **Zusammenfassung**

Die Erfindung lehrt die Verwendung einer Substanz ausgewählt aus der Gruppe bestehend aus "Aminosäuren, Aminosäureanaloge, Zuckerphosphate, Zuckerphosphatanaloge und Mischungen solcher Substanzen" zur Herstellung einer pharmazeutischen Zusammensetzung zur Behandlung von Tumoren und/oder zur Immunsuppression und/oder der Sepsis durch Modulation der Assoziation des Glycolyse-Enzym-Komplexes/M2-PK und/oder durch Hemmung von Transaminasen und/oder durch Auflösung der Bindung der Malat Dehydrogenase an p36.

## Beschreibung

### Gebiet der Erfindung.

Die Erfindung betrifft Verwendungen von Zuckerphosphaten, Zuckerphosphatanalogen, Aminosäuren und/oder Aminosäureanalogen zur Modulation von Stoffwechselprozessen.

Hintergrund der Erfindung.

Verschiedene Erkrankungen gehen einher mit Veränderungen des Zellstoffwechsels. Insbesondere in Tumorgewebe verläuft die Energieerzeugung zumindest zum Teil über andere Mechanismen als bei gesundem Gewebe. Diese tumorspezifischen Mechanismen sind Ansatzpunkte für Tumortherapien, die spezifisch auf das Tumorgewebe wirken, und vergleichsweise geringe Nebenwirkungen haben. Dabei wird selektiv das Tumorwachstum gehemmt und/oder Apoptose von Tumorzellen eingeleitet.

### Stand der Technik.

Es ist bekannt, daß Tumore einem veränderten Metabolismus unterliegen. Dieser veränderte Stoffwechsel führt dazu, daß die Glucose vorrangig für die Nukleinsäuresynthese verwendet wird. Gleichzeitig wird eine neue Energiequelle, die Aminosäure Glutamin, erschlossen. Glutamin Kommt in allen Geweben in hohen Konzentrationen vor. Typischerweise weist Tumorgewebe eine ausgeprägte Hypoxie, i.e. Mangel an Sauerstoff, auf aufgrund des in der Regel desorganisierten Wachstums von Blutgefäßen in dem Tumorgewebe. Dies macht deutlich, daß ein Anpassung an hypoxische Bedingungen ein wesentlicher Schritt im Tumorwachstum ist. Der anaerobe Umsatz von Glukose zwecks Energieerzeugung durch Glykolyse ist daher ein gemeinsames Merkmal der meisten Tumorgewebeaggregate. Bezüglich allgemeiner weiterführender Literatur hierzu wird auf die Literaturstelle C.V. Dang et al., TIBS 24:68-72, 1999, verwiesen. Die Pyruvatkinase (PK) ist ein Schlüsselenzym der Glykolyse und katalysiert die energieliefernde Umwandlung von Phosphoenolpyruvat in Pyruvat. Vier gewebespezifische Isoformen sind bekannt, PK Typ L, R, M1 und M2 (siehe E. Eigenbrodt et al., Critical Reviews in Oncogenesis, Vol. 3, M. Perucho, Ed., CRC-Press, Boca Raton, Florida, Seiten 91 - 115, 1992). M2-PK ist die embryonale Form und ersetzt alle anderen Formen in proliferierenden Zellen und Tumorzellen (siehe G.E.J Staal et al., Biochemical and Molecular Aspects of Selected Cancers, T.G. Pretlow et al., Eds., Academic Press Inc., San Diego, 1, Seiten 313 - 337, 1991, sowie U. Brinck et al., Virchows Archiv 424, Seiten 177 - 185, 1994). M2-PK Protein der Ratte besteht aus 530 Aminosäuren und unterscheidet sich nur in einem Rest von humaner M2-PK (siehe T. Noguchi et al., J. Biol. Chem., 261, Seiten 13807 - 13812, 1986, sowie K. Tani et al, Gene, 73, Seiten 509 - 516, 1988). M2-PK ist ein glykolytisches Emzym, welches in einer hochaktiven tetrameren und einer wenig aktiven dimeren Form vorliegen kann. Nur die hochaktive tetramere Form ist im Glycolyse-Enzym-Komplex assoziiert. Der Gylcolyse-Enzym-Komplex ist eine Assoziation von Glycolyse-Enzymen, NDPK, Adenylatkinase, RNA, A-raf und Komponenten der Proteinkinasekaskade. Der Übergang zwischen beiden Formen der M2-PK reguliert letztendlich den glykolytischen Umsatz in Tumorzellen (siehe S. Mazurek et al.: J. Cell. Physiol. (1996) 167:238-250; S. Mazurek et al.: Anticancer Res. (1998) 18:3275-3282; S. Mazurek et al., J. Bioenerg. Biomembr., 29, Seiten 315-330, 1997). Die Aktivität von M2-PK kontrolliert also den Transit des glykolytischen Pfades. Liegt die M2-PK in der dimeren Form vor, werden die Glucose-Kohlenstoffatome in abzweigende Syntheseprozesse eingeschleust. Liegt die M2-PK als tetramere und im Glycolyse-Enzym-Komplex assoziierter Form vor, wird die Glucose sehr effektiv unter Energiegewinnung zu Pyruvat und Lactat umgesetzt. Die Überexpression von M2-PK erlaubt Zellen das Überleben unter Bedingungen mit niedrigem Sauerstofflevel, da oxidative Phosphorylisierung für die Produktion von ATP durch PK nicht benötigt wird. Generell wird in bösartigen Tumoren und in dem Blut von Tumorpatienten eine erhöhte Menge an M2-PK gefunden.

Aus der Literaturstelle E. Eigenbrodt et al., Biochemical an Molecular Aspects of Selected Cancers, Vol. 2, S. 311 ff., 1994, ist es bekannt, zur Hemmung der Glycolyse Glucoseanaloge einzusetzen. Andere hieraus bekannte Ansätze sind der Einsatz von Inhibitoren glycolytischer Isoenzyme, beispielsweise durch geeignete Komplexbildung oder Inhibierung von Komplexbildungen. Im Ergebnis werden Tumorzellen gleichsam ausgehungert. Problematisch bei den vorstehenden Verbindungen ist, daß viele davon geneotoxisch und/oder nicht hinreichend spezifisch für Tumorzellen sind.

Aus der Literaturstelle Eigenbrodt et al. in Critical Reviews in Oncogenesis (1992) (Perucho, M. ed.) CRC-Press, Boca Raton, Florida; 3:91-115, ist es bekannt, daß Fructose-1,6-bisphosphat zu einer Verschiebung zur hochaktiven tetrameren Form der M2-PK führt, wodurch der glycolytische Flux in Tumorzellen reguliert werden kann. Aus dieser Literaturstelle ist weiterhin bekannt, daß Alanin und Leucin M2-PK inhibieren.

Im Zusammenhang mit einem neuen Wirkstoff gegen inflammatorischen Erkrankungen sowie gegen Autoimmunreaktionen ist es aus der Literaturstelle U. Mangold et al., Eur. J. Biochem., 266:1-9, 1999, bekannt, daß 2-Cyano-3-hydroxyl-but-2-[4-triflouromethyl-phenyl]-amid (folgend Chba) in die Glykolyse eingreift.

Transaminasen sind Enzyme, die bei der Transaminierung Aminogruppen von 2-Aminosäuren auf 2-Ketosäuren übertragen. Es handelt sich um eine Untergruppe der Transferasen. Die prosthetische Gruppe ist Pyridoxalphosphat. Eine Hemmung von Transaminasen führt zu einem Anstieg der Aminosäuren. Aus der Literaturstelle E. Eigenbrodt et al., Biochemical an Molecular Aspects of Selected Cancers, Vol. 2, S. 311 ff., 1994, ist es bekannt, daß Aminooxyacetat und Cycloserin die Glutamat-Pyruvat-Transaminase inhibieren und die Proliferation von Zellen hemmen können.

Technisches Problem der Erfindung.

Der vorliegenden Erfindung liegt das technische Problem zu Grunde, Wirkstoffe anzugeben, welche in der Lage sind, die Proliferation von Krebszellen und somit das Wachstum neoplastischer Tumore zu hemmen sowie überschießende Abwehrreaktionen des Körpers, wie z.B. septischer Schock, Autoimmunerkrankungen, Transplantatabstoßungen sowie akute und chronische Entzündungsreaktionen zu inhibieren, und zwar bei gleichzeitig lediglich geringfügiger bis keiner Zytotoxizität gegenüber normalen Zellen des Blutes, des Immunsystems und normaler Gewebszellen.

Grundzüge der Erfindung.

Zur Lösung dieses technischen Problems lehrt die Erfindung die Verwendung einer Substanz ausgewählt aus der Gruppe bestehend aus "Aminosäuren, Aminosäureanaloge, Zuckerphosphate, Zuckerphosphatanaloge und Mischungen solcher Substanzen" zur Herstellung einer pharmazeutischen Zusammensetzung zur Behandlung von Tumoren und/oder zur Immunsuppression und/oder der Sepsis durch Modulation der Assoziation des Glycolyse-Enzym-Komplexes/M2-PK und/oder durch Hemmung von Transaminasen und/oder durch Auflösung der Bindung der (mitochondralen) Malat Dehydrogenase an p36.

Die Erfindung beruht zunächst auf der Erkenntnis, daß in Tumorzellen das Verhältnis tetramerer und dimerer M2-PK ca. 50 : 50 beträgt. Hieran anschließend wurde erkannt, daß eine Veränderung dieses Verhältnisses, also eine Verschiebung zu einer der beiden Formen, für die Tumortherapie geeignet ist. So wurde gefunden, daß bei einer vollständigen Tetramerisierung der M2-PK die Nukleinsäuresynthese und folglich die Proliferation gehemmt wird. Bei einer vollständigen Dimerisierung kommt es dagegen zu einer Hemmung der Energiegewinnung aus Glucose mit der Folge von Apoptose, ein ebenso positiver therapeutischer Effekt. Überraschenderweise können also beide Effekte für eine Tumortherapie genutzt werden. Dabei sind keine zytotoxische Wirkungen zu erwarten, da dieser Stoffwechselzustand tumorgewebespezifisch ist.

Neben der Veränderung bei der Pyruvat-Kinase-Isoenzym-Ausstattung kommt es bei der Tumorentstehung zu einem Verschwinden der NAD-abhängigen cytosolischen Glycerol 3-Phosphat Dehydrogenase. Dies führt dazu, daß der Wasserstoff aus der glycolytischen Glycerinaldehyd 3-Phosphat Dehydrogenase-Reaktion über den Malat-Aspartat-Shuttle in die Mitochondrien transportiert werden muß. Dies führt zur Aktivierung des Abbaus von Glutamin zu Pyruvat und Lactat (Glutaminolyse). Die Glutaminolyse sichert die Pyruvat- und Energiebereitstellung unter Bedingungen, in der die M2-PK inaktiviert ist. Ein wichtiger Bestandteil des Malat-Aspartat-Shuttles ist die Vorstufe der mitochondrialen Malat Dehydrogenase, die im Cytosol durch die Bindung an das Phosphoprotein p36 gehalten wird. Die Bindung der mitochondrialen Malat Dehydrogenase an p36 im Cytosol kann durch Aminosäuren und durch Zuckerphosphate sowie Analoge hierzu aufgehoben werden.

Es wurde also weiterhin erkannt, daß eine Modulation der Assoziation Glycolyse-Enzym-Komplex/M2-PK auch indirekt, i.e. ohne direkte Bindung eines Wirkstoffes an M2-PK, erfolgen kann. Wenn nämlich die Transaminierung gehemmt wird, und/oder die Bindung der Malat Dehydrogenase an p36 aufgehoben wird, so führt dies wiederum zu einem Anstieg oder Abfall an Aminosäuren, welche ihrerseits mit M2-PK in Wechselwirkung treten und folglich die Assoziation modulieren.

Mit erfindungsgemäß eingesetzten Substanzen lassen sich neben der Glutamat-Pyruvat-Transaminase die Glutamat-Oxalacetat-Tranaminase, die Glutamat-3-Hydroxypyruvat-Transaminase und andere verzweigtkettige α-Ketocarbonsäure-Transaminasen inhibieren.

Der Begriff der Analoge bezeichnet Verbindungen, welche sich strukturell aus natürlichen Aminosäuren bzw. Zuckern herleiten lassen, i.e. von diesen verschieden sind, welche jedoch die gleiche oder eine noch stärkere Modulation der Glycolyse-Enzym-Komplex /M2-PK Assoziation, eine Transaminase-Inhibition und/oder eine Auflösung der p36-Malat Dehydrogenase Bindung bewirken, als die zugrundeliegende natürliche Substanz. Ein Analog kann insbesondere ein Derivat darstellen, i.e. eine natürlicherweise vorliegende Gruppe oder ein H-Atom können durch eine andere, nicht-natürlicherweise vorkommende Gruppe ersetzt sein. Dies betrifft sowohl Seitenketten als auch die Kernstruktur; so kann die Carboxylgruppe einer Aminosäure insbesondere durch eine Cyanidgruppe ersetzt sein. Im Falle der Zuckerphosphate kann eine oder mehrere Phosphatgruppen durch eine Cyanidgruppe ersetzt sein. Aminosäurenanaloge sind insbesondere auch die Vorläufer der Aminosäuren, die α-Ketosäuren, sowie solche α-Ketosäuren, bei welchen die -COOH Gruppe durch eine Cyanidgruppe (-CN) ersetzt ist.

Bevorzugte Ausführungsformen der Erfindung.

Im Rahmen der Erfindung sind diverse, nicht beschränkende Ausführungsformen möglich. So kann eine erfindungsgemäße pharmazeutische Zusammensetzung mehrere verschiedene erfindungsgemäß eingesetzte Verbindungen enthalten. Weiterhin kann eine erfindungsgemäße pharmazeutische Zusammensetzung zusätzlich einen von einem erfindungsgemäß eingesetzten Wirkstoff verschiedenen Wirkstoff enthalten. Dann handelt es sich um ein Kombinationspräparat. Dabei können die verschiedenen eingesetzten Wirkstoffe in einer einzigen Darreichungsform präpariert sein, i.e. die Wirkstoffe sind in der Darreichungsform gemischt. Es ist aber auch möglich, die verschiedenen Wirkstoffe in räumlich getrennten Darreichungsformen gleicher oder verschiedener Art herzurichten.

Hinsichtlich des erfindungsgemäß eingesetzten Wirkstoffes ist es möglich, daß die Substanz ausgewählt ist aus der Gruppe bestehend aus "Serin, Cycloserin, Valin, Leucin, Isoleucin, Prolin, Methionin, Cystein, Aminoisobutyrat, Aminooxyacetat, Chba, Fructose-1,6-bisphosphat, Glycerat-2,3-bisphosphat, Glycerat-3-phosphat, Ribose-1,5-bisphosphat, Ribulose-1,5-bisphosphat, Analoge solcher Verbindungen und Mischungen solcher Substanzen.

Die Substanz ist bevorzugterweise ausgewählt aus der Gruppe bestehend aus Verbindungen der Formel I und Mischungen solcher Verbindungen, 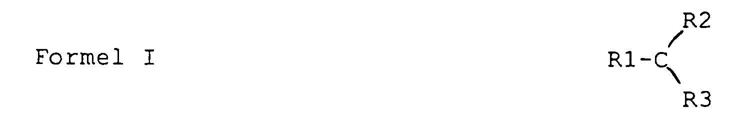 wobei R1 = -NR4R5 oder ein Aminosäurenrest, ggf. derivatisiert, ist, R2 = -COOH, -CN oder -NR4R5, wobei R4 und R5 gleich oder verschieden und H, C1-C18-Alkyl, -Aryl, oder -Aralkyl, ggf. substituiert mit -J, -Cl, und/oder -F, sein können, R3 = =O, ist.

Es handelt sich bei diesen besonders bevorzugten Stoffen typischerweise um 2- bzw. α-Oxonitrile bzw. Ketosäuren (ggf. verestert). Diese Substanzen sind Aminosäureanaloge mit hoher Wirksamkeit.

Im Zusammenhang mit Cycloalkyl- und Aryl-Gruppen ist anzumerken, daß hiermit sowohl homo- als auch heteroatomare aromatische Gruppen bezeichnet sind. Beispiele für heterocyclische Gruppen sind: Furanyl, Thiophenyl, Pyrrolyl, Isopyrrolyl, 3-Isopyrrolyl, Pyrazolyl, 2-Isoimidazolyl, Triazolyl, Oxazolyl, Isoaxazolyl, Thiazolyl, Isothiazolyl, Pyridyl, Pyrazinyl, Pyrimidinyl, Pyridazinyl, Piperazinyl, Triazinyl, Oxazinyl, Indenyl, Benzofurnyl, Benzothiofuranyl, Indolyl, Isoindazolyl, Benzoxazolyl, wobei die genannten Gruppen teilweise hydriert sein können.

Folgend werden beispielhafte Verbindungen angegeben.

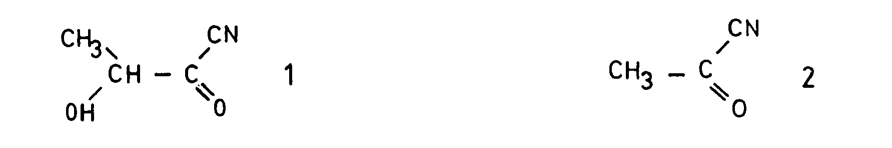

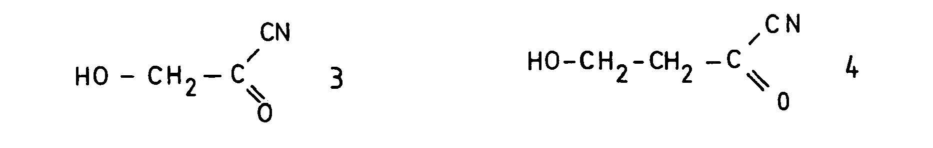

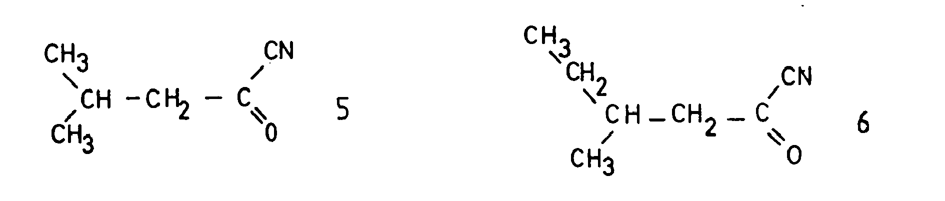

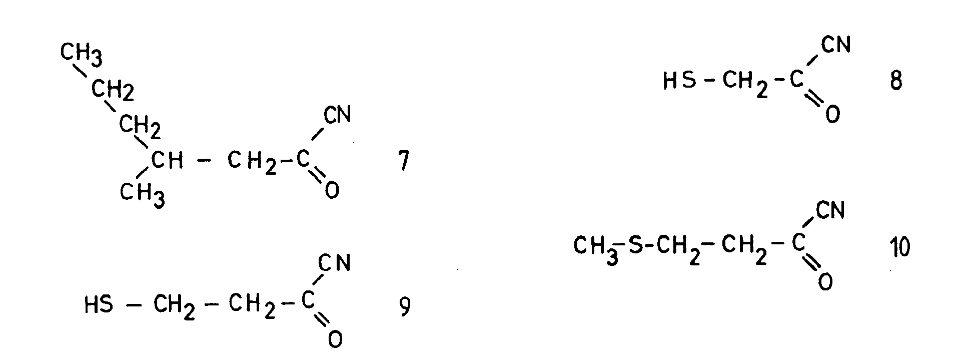

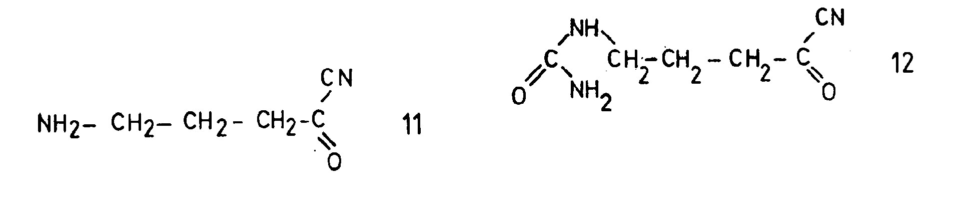

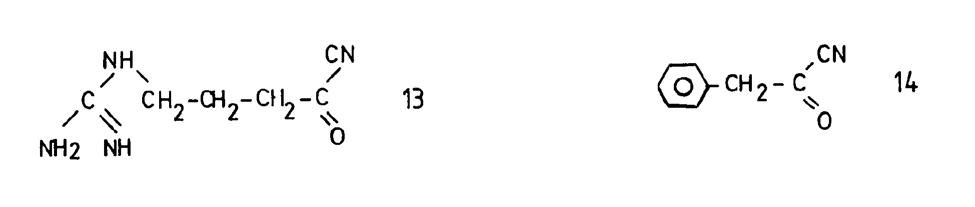

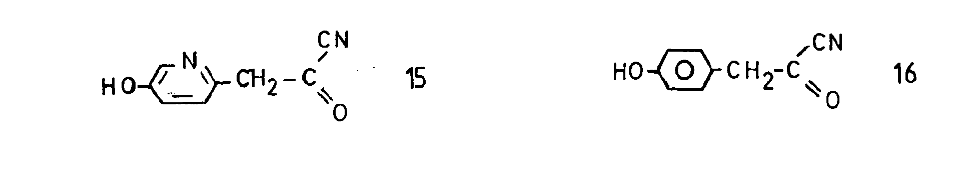

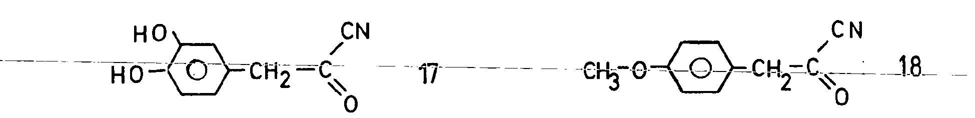

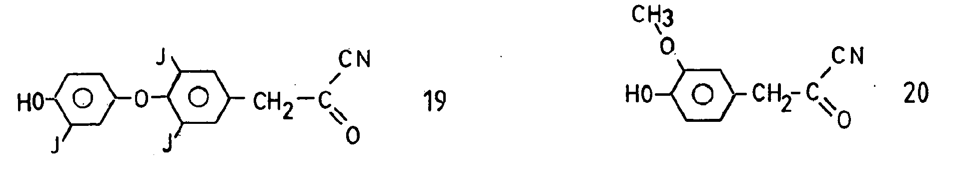

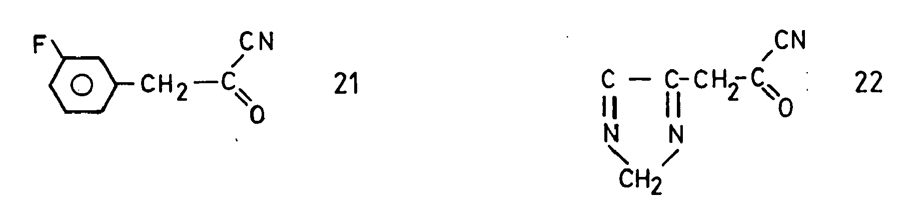

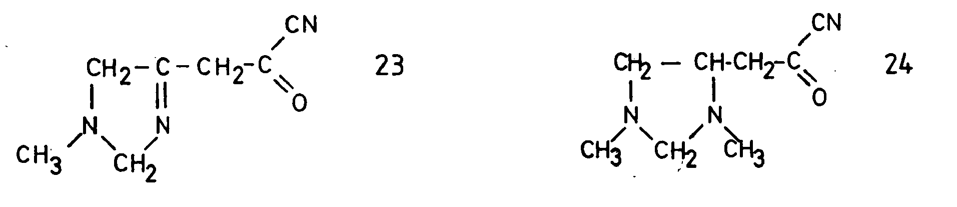

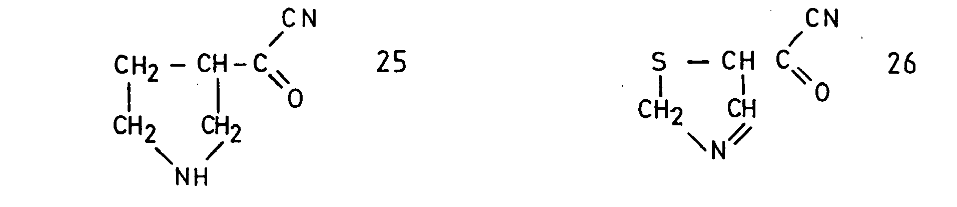

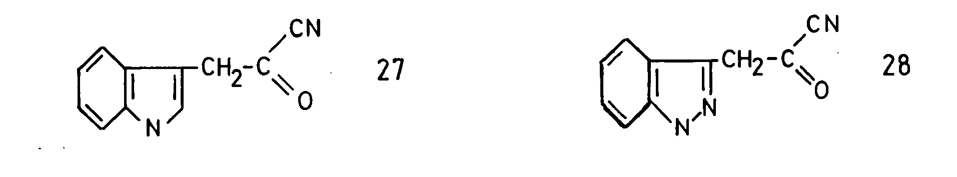

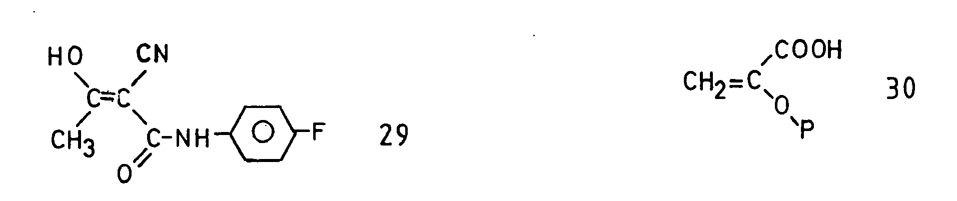

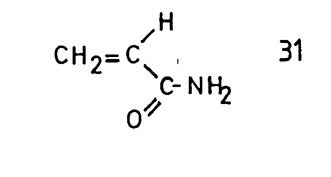

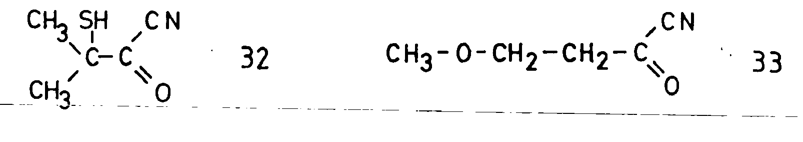

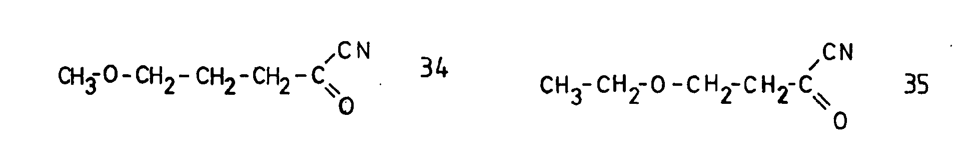

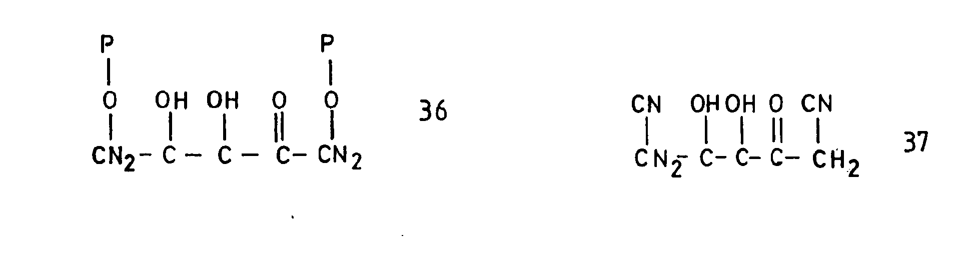

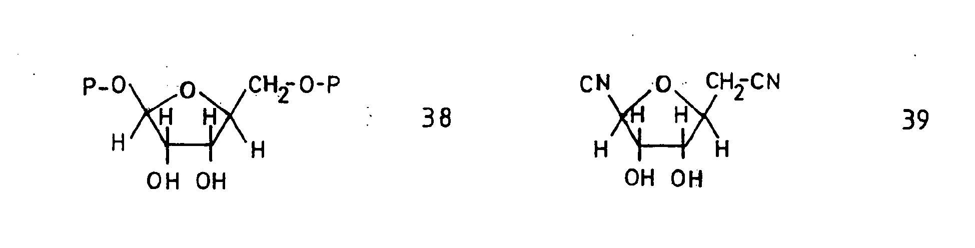

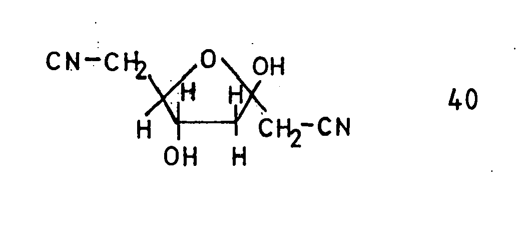

Als Gegenionen für ionische Verbindungen nach Formel I kommen beispielsweise Na⁺, K⁺, Li⁺, und/oder Cyclohexylammonium in Frage.

Die mit erfindungsgemäß verwendeten Verbindungen hergestellten Arzneimittel können oral, intramuskulär, periartikulär, intraartikulär, intravenös, intraperitoneal, subkutan oder rektal verabreicht werden. Besonders bevorzugt ist jedoch die intravenöse Verabreichung, insbesondere im Falle vom Chba, Aminooxyacetat (NH₂-CO-COOH) oder Zuckerphosphaten bzw. Zuckerphosphatanalogen.

Die Erfindung lehrt weiterhin ein Verfahren zur Herstellung eines Arzneimittels, das dadurch gekennzeichnet ist, daß mindestens eine erfindungsgemäß verwendete Verbindung mit einem pharmazeutisch geeigneten und physiologisch verträglichen Träger und gegebenenfalls weiteren geeigneten Wirk-, Zusatz- oder Hilfsstoffen mischt und zu der gewünschen Darreichungsform herrichtet.

Geeignete feste oder flüssige galenische Zubereitungsformen sind beispielsweise Granulate, Pulver, Dragees, Tabletten, (Mikro) Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel wie Trägerstoffe, Spreng-, Binde-, Überzugs-, Quellungs-, Gleit- oder Schmiermittel, Geschmacksstoffe, Süßungsmittel und Lösungsvermittler, Verwendung finden.

Als Hilfsstoffe seien Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuß-oder Sesamöl, Polyethylenglykole und Lösungsmittel, wie etwa steriles Wasser und ein- oder mehrwertige Alkohole, z.B. Glycerin, genannt.

Vorzugsweise werden die Arzneimittel in Dosierungseinheiten hergestellt und verabreicht, wobei jede Einheit als aktiven Bestandteil eine bestimmte Dosis der erfindungsgemäßen Verbindung gemäß Formel I enthält. Bei festen Dosierungseinheiten wie Tabletten, Kapseln, Dragees oder Suppositorien kann diese Dosis 1 bis 5000 mg, bevorzugt 50 bis 1000 mg, und bei Injektionslösungen in Ampullenform 1 bis 5000 mg, vorzugsweise 50 bis 2000 mg, bei intramuskulärer Injektion bzw. 1 bis 100 mMol, vorzugsweise 10 bis 100 mMol, bei intraperitonealer Injektion betragen. Für die i.v. Applikation sind entsprechende Dosen, vorzugsweise allerdings um einen Faktor 0,5 bis 0,1 erniedrigt, einsetzbar.

Für die Behandlung eines erwachsenen, 50 bis 100 kg schweren, beispielsweise 70 kg schweren, Patienten sind Tagesdosen von 20 bis 5000 mg Wirkstoff, vorzugsweise 500 bis 3000 mg, indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Im Foglenden wird die Erfindung anhand von lediglich Ausführungsformen darstellenden Beispielen näher erläutert.

### Beispiel 1: Tumormodell

Als Tumormodell wurde die immunkompetente erwachsene Ratte verwendet, die durch i.v.-Infusion therapiert wird. Dies ist ein der Therapie beim Menschen ähnlicheres Modell als die nicht immunkompetente Nacktmaus, die üblicherweise eingesetzt wird. Die Tierversuche waren gemäß §8 Abs. 1 des Deutschen Tierschutzgesetzes genehmigt und wurden entsprechend den Empfehlungen der Tierärztlichen Vereinigung für Tierschutz e.V. durchgeführt. Als Tumorempfänger wurden männliche Inzuchtratten (Sprague-Dawley, 200-250 g, Charles River, Sulzfeld, Deutschland) eingesetzt.

Als Tumorzellen wurde das Novikoff-Hepatom eingesetzt. Unter mehreren getesteten experimentell erzeugten Tumoren erfüllt das Novikoff-Hepatom die Voraussetzungen eines soliden Tumors, der alle Zeichen der Malignität aufweist und morphologisch dem hepatozellulären Karzinom des Menschen ähnelt, am besten. Das Novikoff-Hepatom wurde von Alex B. Novikoff 1951 durch Fütterung einer Diät mit 0,06% 4-Dimethylazobenzol (Buttergelb) auf weiblichen Sprague-Dawley-Ratten induziert [Novikoff B. A transplantatble rat liver tumor induced by 4-dimethylaminoazobenzene. Cancer Res 1951; 17:1010]. Dieser Lebertumor, der sowohl als Aszitestumor als auch als solider Tumor wächst, zeigt die typischen Malignitätskriterien wie Hyperchromasie, Polymorphie, erhöhte Mitoserate und eine zugunsten des Kerns verschobene Kern-Plasma-Relation. In den Tumorzellen erscheint das Chromatingerüst unregelmäßig und die Kerne gelappt, rund oder oval.

Die Novikoff-Hepatom-Zellen wurden vom Deutschen Krebsforschungszentrum in Heidelberg zur Verfügung gestellt. Die Zellen wurden in Hank's Lösung aufgenommen und steril einer Sprague-Dawley-Ratte zur Passagierung intraperitoneal injiziert. Innerhalb einer Woche bildete sich ca. 50 ml hämorrhagischer Aszites, der steril entnommen wurde. Die Aufbereitung des nach Zentrifugieren bei 1300 U/min für fünf Minuten in einem Falcon-Röhrchen entstandenen Pellets erfolgte zur weiteren Reinigung der Zellen von anderen Aszitesbestandteilen durch Waschen mit 50 ml Dulbecco's MEM (Gibco BRL, Eggenstein) und Zentrifugation bei 1300 U/min für fünf Minuten. Der Überstand wurde dekantiert und das Pellet in Dulbecco's + 40% fötalem Kälberserum (FKS) gemischt. Nun wurden je 0,7 ml Zellsuspension und 0,7 ml Einfriermedium in Nunc-Röhrchen gefüllt, luftdicht verschlossen, für fünf Minuten bei -20°C und für 12 Stunden bei -80°C vorgekühlt und anschließend in flüssigem Stickstoff tiefgefroren. Das Einfriermedium bestand aus 40% Dulbecco's, 40% FKS und 20% DMSO.

Für die Applikation wurden die Zellen folgendermaßen vorbereitet: nach dem Auftauen wurde das Pellet in einem Falcon-Röhrchen mit 50 ml eines auf 37°C vorgewärmten Mediums (Dulbecco's + 40% FKS) versetzt und für fünf Minuten bei 1300 U/min zentrifugiert. Der Überstand wurde verworfen, der Vorgang nochmals wiederholt.

Nach Zentrifugation und Dekantieren des Überstandes wurde das Pellet mit HBSS aufgefüllt, 100 Mikroliter mit einer Eppendorf-Pipette entnommen und zur Bestimmung der Zahl vitaler Zellen nach Vitalfärbung mit Erythrosin (Fa. BioMed, München, Deutschland) in einer Neubauer-Zählkammer ausgezählt. Die Zellsuspension wurde nach Zentrifugierung und Dekantierung so lange mit HBSS verdünnt, bis die Suspension 5 x 10⁶ vitale Zellen pro ml enthielt. 1 ml dieser Suspension wurde in einer Insulinspritze aufgenommen und der Ratte am Rücken subkutan injiziert.

Hierzu wurde eine rasierte und mit 70%-igem Alkohol desinfizierte Hautfalte des mit Äther narkotiserten Tieres angehoben und mit einer Kanüle Nr. 14 in Längsrichtung von kaudal nach kranial durchstochen und die Tumorzellen subkutan injiziert.

### Beispiel 2: Behandlung

Die Infusion der Versuchstiere mit erfindungsgemäßen Substanzen begann, sobald der Tumor ein Volumen von 1 ml erreichte. Die Tumorgröße wurde durch CT-gestützte Volumimetrie bestimmt. Hierfür wurden die Ratten mit 0,315 mg Fentanylzitrat/kg Körpergewicht i.m. (Hypnorm®, Janssen, Beersee, Belgien) betäubt. Mit einem Somatom Plus 4-Scanner (Siemens, Erlangen, Deutschland) wurde ein Spiral-CT mit einer Schichtdicke von 2 mm, einem Pitch von 1,5 und 2mm Inkrement bei 120 kVp mit 320 mAs durchgeführt. Ein Weichgewebsalgorithmus wurde verwendet.

Bei einer zu behandelnden Ratte wurde ein Silikonschlauch (SilasticR 0,012 inch x 0,025 inch, Nr.602-105 HH 061999, Dow Corning Corp., Midland, Mich., USA) mit Hilfe von Chloroform auf das Ende eines 5 cm langen spiralförmigen Stückes PE 10 (Polyäthylen)-Katheter (Clay Adams, Parsippany, NJ, USA) aufgeschoben. Das gegenüberliegende Ende wurde mit einem 30 cm langen Stück PE 20-Katheter verschmolzen. Das Silikonstück wurde in die linke Jugularvene des Empfängers eingebracht und mit einer Ligatur gesichert, wie vormals beschrieben [Weeks JR. Long term intravenous infusion. In: Meyers RD (Hrsg.) Methods in Psychobiology, Academic Press 1972;2:155]. Der spiralförmige Katheteranteil kam im Subkutangewebe zu liegen und sorgte für die notwendige Extralänge, um bei Kopfbewegungen des Tieres eine Katheterdislokation zu vermeiden. Das andere Ende wurde durch die Haut des Rückens nach außen geleitet, in einem Metallspiralschlauch, der über ein Mieder am Tier befestigt wurde, geschützt und mit einer Infusionspumpe verbunden, die eine körpergewichtsadaptierte kontinuierliche Infusion ermöglichte. Die Tiere befanden sich während der Infusion in einem metabolischen Käfig.

Jeweils 10 randomisierten Tieren pro Gruppe wurden ab einem Tumorvolumen von 1 ml kontinuierlich über 10 Tage mit der jeweiligen Substanz (1.25mM Aminooxyacetat oder 10 µM Chba) appliziert. Kontrolltiere erhielten eine isovolumische Menge an NaCl. Alle Tiere hatten freien Zugang zu Wasser und R3-EWOS-ALAB-Zuchtfutter (ALAB, Sollentuna, Schweden). Nach 10 Tagen wurden die Tiere mit 0,315 mg Fentanylzitrat/kg Körpergewicht i.m. (HypnormR, Janssen, Beersee, Belgien) betäubt, der Tumor entnommen und sein Volumen an Hand der verdrängten Wassermenge bestimmt.

### Beispiel 3: Ergebnisse.

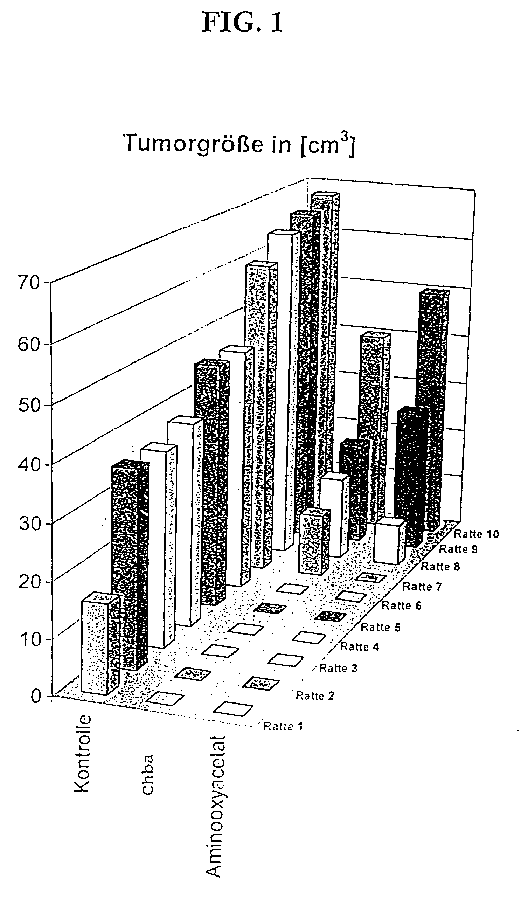

Die Figur 1 zeigt die erhaltenen Ergebnisse. Während die Kontrolltiere Tumor von beachtlicher Größe aufwiesen, konnte mit Chba, bzw. Aminooxyacetat eine beachtliche Hemmung des Tumorwachstums festgestellt werden. Bei Behandlungsbeginn mit relativ kleiner Tumorgröße konnte sogar eine praktisch vollständige Hemmung des Tumorwachstums, sogar Apoptose, beobachtet werden.

### Beispiel 4: Dosisabhängigkeit der Proliferationshemmung.

In diesem Beispiel wird die Dosisabhängigkeit der Proliferationshemmung für verschiedene erfindungsgemäß eingesetzte Verbindungen dargestellt.

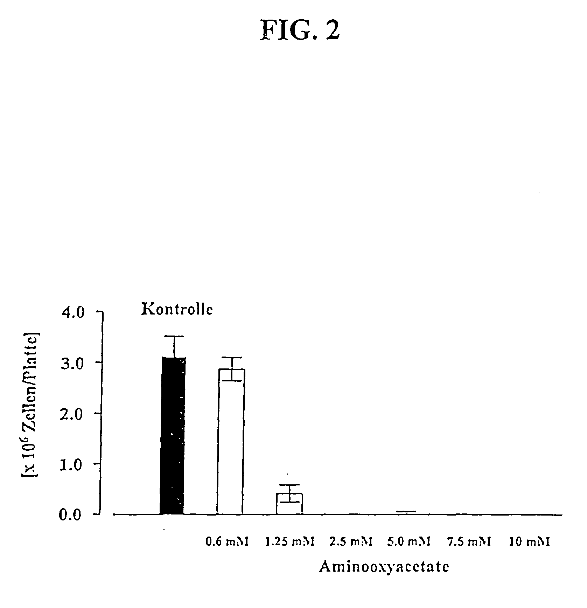

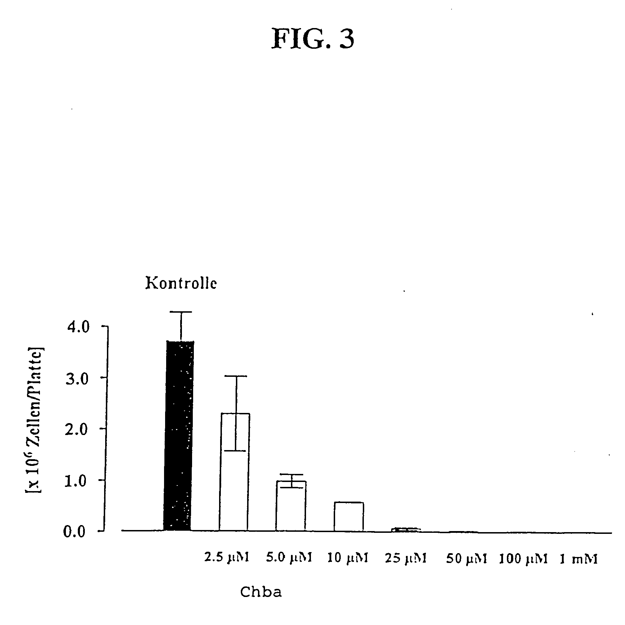

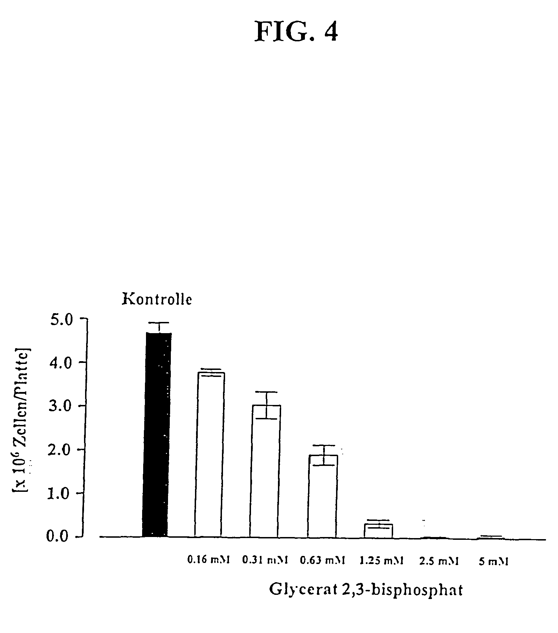

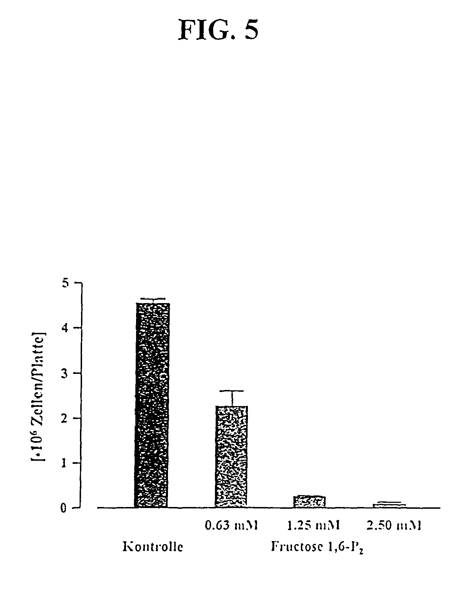

Für die Messungen wurden Novikoff-Hepätomzellen auf übliche Weise kultiviert. Die Kontrollgruppe erhielt Lösungsmittel ohne Wirksubstanz. Die weiteren Gruppen erhielten verschiedene Dosen der jeweiligen Verbindung. Nach vier Kultivierungstagen mit bzw. ohne Wirkstoff wurden die Zelldichte auf übliche Weise bestimmt. In der Figur 2 ist die Dosisabhängigkeit der erhaltenen Zelldichten für Aminooxyacetat, in der Figur 3 für Chba, in der Figur 4 für Glycerat 2,3-bisphosphat und in der Figur 5 für Fructose-1,6-bis- phosphat dargestellt. Man erkennt in allen Fällen eine praktisch vollständige Hemmung jedenfalls bei höheren Dosen.

## Patentansprüche

1. Verwendung einer Substanz ausgewählt aus der Gruppe bestehend aus "Aminosäuren, Aminosäureanaloge, Zuckerphosphate, Zuckerphosphatanaloge und Mischungen solcher Substanzen" zur Herstellung einer pharmazeutischen Zusammensetzung zur Behandlung von Tumoren und/oder zur Immunsuppression und/oder der Sepsis durch Modulation der Assoziation des Glycolyse-Enzym-Komplexes/M2-PK und/oder durch Hemmung von Transaminasen und/oder durch Auflösung der Bindung der Malat Dehydrogenase an p36.

2. Verwendung nach Anspruch 1, wobei die Substanz ausgewählt ist aus der Gruppe bestehend aus "Serin, Cycloserin, Valin, Leucin, Isoleucin, Prolin, Methionin, Cystein, Aminoisobutyrat, Aminooxyacetat, Chba, Fructose-1,6-bisphosphat, Glycerat-2,3-bisphosphat, Glycerat-3-Phosphat, Ribose-1,5-bisphosphat, Ribulose-1,5-bisphosphat, Analoge solcher Verbindungen und Mischungen solcher Substanzen".

3. Verwendung nach Anspruch 1, wobei die Substanz ausgewählt ist aus der Gruppe bestehend aus Verbindungen der Formel I und Mischungen solcher Verbindungen, 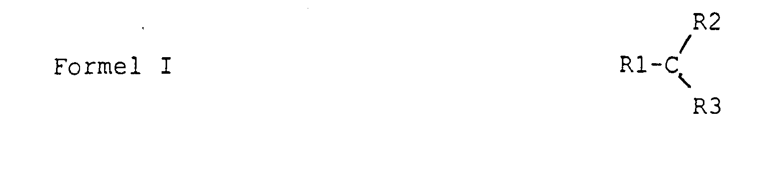 wobei R1 = -NR4R5 oder ein Aminosäurenrest, ggf. derivatisiert, ist,
wobei R2 = -COOH, -CN oder -NR4R5,
wobei R4 und R5 gleich oder verschieden und H, C1-C18-Alkyl, -Aryl, oder -Aralkyl, ggf. substituiert mit -J, -C1, und/oder -F, sein können, und
wobei R3 = =O,

4. Verwendung nach einem der Ansprüche 1 bis 3, wobei die pharmazeutische Zusammensetzung zur i.v. Applikation hergerichtet ist.

5. Verwendung nach einem der Ansprüche 1 bis 4, wobei die pharmazeutische Zusammensetzung für Darreichung in einer Tagesdosis von 0,1 bis 80 mg je kg Körpergewicht hergerichtet ist.
